(19) Europäisches Patentamt
European Patent Office
Office européen des brevets

(11) Veröffentlichungsnummer: **0 098 541**
**B1**

(12) # EUROPÄISCHE PATENTSCHRIFT

(45) Veröffentlichungstag der Patentschrift: **04.06.86**

(51) Int. Cl.⁴: **C 07 C 148/00,** C 07 C 149/10, C 07 C 149/30

(21) Anmeldenummer: **83106413.4**

(22) Anmeldetag: **01.07.83**

(54) **Verfahren zur Herstellung von Thioethern.**

(30) Priorität: **09.07.82 DE 3225709**

(43) Veröffentlichungstag der Anmeldung: **18.01.84 Patentblatt 84/3**

(45) Bekanntmachung des Hinweises auf die Patenterteilung: **04.06.86 Patentblatt 86/23**

(84) Benannte Vertragsstaaten: **BE DE FR GB**

(56) Entgegenhaltungen: **DE - B - 1 793 252**

**HOUBEN-WEYL "Methoden der Organischen Chemie" 4. Auflage, Band 9 1955, GEORG THIEME VERLAG, Stuttgart Selten 117-118**

(73) Patentinhaber: **BASF Aktiengesellschaft, Carl-Bosch-Strasse 38, D-6700 Ludwigshafen (DE)**

(72) Erfinder: **Bott, Kaspar, Dr., Rieslingweg 4, D-6706 Wachenheim (DE)**

**EP 0 098 541 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Thioethern durch Umsetzung von Alkalimercaptiden mit Alkoholen und Kohlenmonoxid oder mit Formiaten.

Eine häufig verwendete Synthese zur Herstellung von Dialkylthioethern oder Alkylarylthioethern besteht darin, dass man die Alkalisalze von aliphatischen oder aromatischen Mercaptanen mit den gebräuchlichen Alkylierungsmitteln, z. B. Alkylhalogeniden, Alkylsulfaten bzw. Alkylsulfonaten zur Umsetzung bringt (Houben-Weyl, Methoden der Organischen Chemie, Band IX, Seiten 97–116). Diese Verfahren haben jedoch den Nachteil, dass man die genannten Alkylierungsmittel vielfach zuerst aus den entsprechenden Alkanolen herstellen muss.

Es ist auch bekannt, Thiophenole mit Ethanol über Thorium- oder Aluminiumkatalysatoren bei 350–450°C zu Ethylarylthioethern umzusetzen. Thioether werden auch durch Umsetzung von Phenylcarbinolen mit Thioglykolsäure in Gegenwart von Salzsäure oder mit p-Thiokresol in Gegenwart von Ameisensäure in der Wärme hergestellt. Ebenfalls kann man Phenylcarbinole mit Thiolen in Gegenwart von Chlorwasserstoff oder Bortrifluorid umsetzen oder tert.-Butylalkohol mit Thioglykolsäure in salzsaurer Lösung kondensieren (Houben-Weyl, loc. cit., Seiten 117–118). Diese Reaktionen ergeben aber nur mit Alkoholen mit einer besonders leicht austauschbaren Hydroxylgruppe befriedigende Ausbeuten. Mit weniger reaktionsfähigen Alkoholen wie Methanol oder Ethanol kann man die Alkylierung der Mercaptane erst bei Temperaturen oberhalb 350°C in vorgenannter Weise durchführen. Ein derartiges Verfahren schränkt die Auswahl der an der Reaktion beteiligten Ausgangsstoffe stark ein, weil diese nicht nur leicht verdampfbar, sondern auch bei den hohen Temperaturen stabil sein müssen.

Alle vorgenannten Verfahren sind im Hinblick auf einfachen und wirtschaftlichen Betrieb zusammen mit guter Ausbeute und Reinheit des Endstoffs unbefriedigend.

Es wurde nun gefunden, dass man Thioether der Formel

$$R^1{-}S{-}R^2 \qquad I,$$

worin $R^1$ und $R^2$ gleich oder verschieden sein können und jeweils einen aliphatischen, cycloaliphatischen oder araliphatischen Rest bedeuten, $R^1$ auch einen aromatischen Rest bezeichnet, durch Umsetzung von Mercaptanen mit Sauerstoffverbindungen vorteilhaft erhält, wenn man Alkalimercaptide der Formel

$$R^1{-}S{-}M \qquad II,$$

worin $R^1$ die vorgenannte Bedeutung besitzt und M ein Alkaliatom bezeichnet,

a) mit Alkoholen der Formel

$$R^2{-}OH \qquad III,$$

worin $R^2$ die vorgenannte Bedeutung besitzt, und Kohlenmonoxid oder

b) mit Formiaten der Formel

$$H{-}\overset{\overset{\displaystyle O}{\|}}{C}{-}OR^2 \qquad IV,$$

worin $R^2$ die vorgenannte Bedeutung besitzt, umsetzt.

Die Umsetzung kann im Falle der Verwendung von Kalium-2-naphthylmercaptid und Methanol durch die folgenden Formeln wiedergegeben werden:

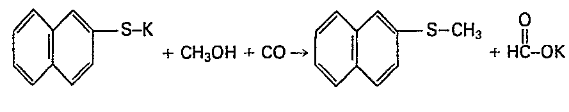


Im Vergleich mit den bekannten Verfahren liefert das Verfahren nach der Erfindung eine grosse Zahl von Thioethern auf einfacherem und wirtschaftlicherem Wege in besserer Ausbeute. Zusätzliche Katalysatoren oder Säuren brauchen nicht verwendet zu werden. Alle diese vorteilhaften Ergebnisse sind im Hinblick auf den Stand der Technik überraschend. Bei der erfindungsgemässen Umsetzung wird ein Teil des Alkohols III durch Kohlenmonoxid-Anlagerung in das entsprechende Formiat IV umgewandelt. Eine solche Nebenreaktion beeinträchtigt überraschend aber nicht die Wirtschaftlichkeit der erfindungsgemässen Thioetherherstellung, da das Formiat IV sich ebenfalls mit dem Mercaptid II zum Thioether I umsetzt.

Die Umsetzung wird mit den Ausgangsstoffen II, IV und III und Kohlenmonoxid in stöchiometrischer Menge oder im Überschuss jedes Ausgangsstoffs zum andern, vorteilhaft in einer Menge von 1 bis 30, insbesondere von 5 bis 15 Mol Ausgangsstoff III oder von 1 bis 20, insbesondere von 5 bis 10 Mol Ausgangsstoff IV je Mol Ausgangsstoff II, durchgeführt. Bevorzugte Ausgangsstoffe II, III und IV und dementsprechend bevorzugte Endstoffe I sind solche, in deren Formeln $R^1$ und $R^2$ gleich oder verschieden sein können und jeweils einen Alkylrest mit 1 bis 18, vorzugsweise 1 bis 12 Kohlenstoffatomen, einen Cycloalkylrest mit 5 bis 12 Kohlenstoffatomen, einen Aralkylrest mit 7 bis 12 Kohlenstoffatomen bedeuten, $R^1$ auch einen Alkylarylrest mit 7 bis 12 Kohlenstoffatomen, einen Naphthylrest oder einen Phenylrest bezeichnet, M für ein Kalium- oder Natriumatom steht. Die vorgenannten Reste können noch durch unter den Reaktionsbedingungen inerte Gruppen, z. B. Alkylgruppen, Alkylthiogruppen oder Alkoxygruppen mit jeweils 1 bis 4 Kohlenstoffatomen, substituiert sein.

So sind beispielsweise die folgenden Alkalimercaptide als Ausgangsstoffe II geeignet: Das Kalium- oder Natriummercaptid von Methyl-, Ethyl-, Propyl-, Isopropyl-, Butyl-, Isobutyl-, sek.-Butyl-, tert.-Butyl-, Pentyl-, Isopentyl-, Hexyl-, Heptyl-, Octyl-, Nonyl-, Decyl-, Undecyl-, Dodecyl-, Stearyl-, Benzyl-, Cyclohexyl-, Cyclopentyl-, Phenyl-, o-, m-, p-Methylphenyl-, 1-Naphthyl-, 2-Naphthyl-mercaptan.

Folgende Alkohole und Formiate sind z. B. als Ausgangsstoffe III bzw. IV geeignet: Methyl-, Ethyl-, Propyl-, Isopropyl-, Butyl-, Isobutyl-, sek.-Butyl-, Pentyl-, Isopentyl-, Hexyl-, Heptyl-, Octyl-, Nonyl-, Decyl-, Undecyl-, Dodecyl-, Stearyl-, Benzyl-, Cyclohexyl-, Cyclopentyl-alkohol; Ester der Ameisensäure mit vorgenannten Alkoholen.

Die Umsetzung wird zweckmässig bei einer Temperatur von 100 bis 200, vorzugsweise von 120 bis 180°C, insbesondere 140 bis 160°C, und einem Druck von 50 bis 400, vorzugsweise 100 bis 320 bar, kontinuierlich oder diskontinuierlich durchgeführt. Die vorgenannten Reaktionsdrücke werden durch Zugabe von Kohlenmonoxid zum Reaktionsgemisch bei der entsprechenden Reaktionstemperatur eingestellt, sie bestehen somit aus dem Partialdruck des Reaktionsgemisches und dem Partialdruck des CO. Verwendet man Gasgemische, so setzen sich die vorgenannten Reaktionsdrücke nur aus den Partialdrücken des Reaktionsgemisches und des CO zusammen, wobei der Partialdruck der weiteren Gase noch dazutritt, z. B. sich dann ein Gesamtdruck von zweckmässig 100 bis 500, vorzugsweise 150 bis 420 bar, einstellt. Im allgemeinen dienen die Ausgangsstoffe, insbesondere der Alkohol III oder das Formiat IV, als Reaktionsmedium, doch können auch zusätzlich organische Lösungsmittel, z. B. Ether wie Tetrahydrofuran, Dioxan, Diethylether, aromatische Kohlenwasserstoffe wie Benzol, Toluol, Xylole, zweckmässig in Mengen von 100 bis 2000 Gew.-% zusätzlichem Lösungsmittel, bezogen auf Ausgangsstoff II, eingesetzt werden. Verwendet man Ausgangsstoff III bzw. IV auch als Lösungsmittel des Ausgangsgemisches, kommen zweckmässig 100 bis 2000, insbesondere 200 bis 600 Gew.-% Ausgangsstoff III bzw. IV, bezogen auf die Gewichtsmenge Ausgangsstoff II, zusätzlich zu den vorgenannten, für die Umsetzung verwendeten bzw. vorteilhaften Mengen an Ausgangsstoff III bzw. IV in Betracht.

Die Reaktion kann wie folgt durchgeführt werden: Ein Gemisch der Ausgangsstoffe II, III und Kohlenmonoxid bzw. der Ausgangsstoffe II und IV wird während 8 bis 20 h bei der Reaktionstemperatur und dem Reaktionsdruck gehalten. Zweckmässig gibt man zunächst die Ausgangsstoffe bei tiefer Temperatur, z. B. 20 bis 50°C, zusammen, gibt Kohlenmonoxid so lange zu, bis sich der Reaktionsdruck einstellt, erhitzt unter Druck dann das Gemisch während 15 bis 30 min bis zur Reaktionstemperatur und hält das Gemisch dann während vorgenannter Reaktionszeit bei der Reaktionstemperatur und dem Reaktionsdruck, wobei der Druck durch laufende, den Verbrauch ersetzende Mengen an Kohlenmonoxid aufrechterhalten wird. Gegebenenfalls kann auch der Reaktionsdruck durch Gemische von Kohlenmonoxid und inerten Gasen, z. B. Stickstoff, eingestellt und/oder gehalten werden. Aus dem Reaktionsgemisch wird dann der Endstoff in üblicher Weise, z. B. durch Destillation, abgetrennt.

In einer bevorzugten Ausführungsform wird die Herstellung des Alkalimercaptids aus dem Mercaptan und die erfindungsgemässe Umsetzung einbadig durchgeführt. Man gibt zweckmässig das dem Ausgangsstoff II zugrundeliegende Mercaptan, den Alkohol III bzw. das Formiat IV und ein Alkalialkoholat bei tiefer Temperatur, zweckmässig von 10 bis 20°C, zusammen und führt dann die Erhitzung und erfindungsgemässe Umsetzung unter Druck und weiterhin die Abtrennung des Endstoffs I in vorgenannter Weise durch. Die Mengenverhältnisse von Mercaptan zu den Ausgangsstoffen III bzw. IV entsprechen zweckmässig den vorgenannten Mengenverhältnissen von Mercaptid II zu den Ausgangsstoffen III bzw. IV. Als Alkalialkoholat, zweckmässig Natrium- oder Kaliumalkoholat, wird vorteilhaft das dem Alkohol III bzw. dem im Formiat IV veresterten Alkohol entsprechende Alkoholat verwendet. Das Alkalialkoholat ist bevorzugt ein Alkalialkanolat mit 1 bis 18 Kohlenstoffatomen und wird zweckmässig in Gestalt einer 5- bis 25-gewichtsprozentigen Lösung in dem verwendeten Alkohol III eingesetzt. Mengen von 1 bis 3, insbesondere 1 bis 1,5 Mol Alkalialkoholat je Mol Mercaptan bzw. herzustellenden Ausgangsstoff II sind vorteilhaft.

Die nach dem Verfahren der Erfindung herstellbaren Thioether sind wertvolle Ausgangsstoffe für die Herstellung von Farbstoffen, Pflanzenschutzmitteln, Pharmazeutika und Kautschukhilfsmitteln. Bezüglich der Verwendung wird auf vorgenannte Veröffentlichungen und Ullmans Encyklopädie der technischen Chemie, Band 12, Seiten 292–298, verwiesen.

Beispiel 1

In einem 0,3l-Rührautoklaven aus Edelstahl löste man 37,9 g (0,26 Mol) n-Octyl-1-mercaptan und 20,3 g (0,29 Mol) Kaliummethylat in 120 ml Methanol (3,0 Mol) bei 20°C auf, erhitzte die so gebildete Kalium-octan-1-thiolat-Lösung unter einem Kohlenmonoxid-Druck von 300 bar während 20 min von 20 auf 150°C und hielt das Gemisch dann während 15 h bei 150°C. Das während der Reaktion verbrauchte Kohlenmonoxid wurde laufend ergänzt, wobei der Druck stets auf 300 bar gehalten wurde. Aus dem Reaktoraustrag wurden durch Destillation bei Normaldruck ein Methanol/Methylformiat-Gemisch abgetrennt, und 38,2 g (92% der Theorie) n-Octyl-methylsulfid bei 110°C/34 mbar erhalten.

Beispiel 2

In eine Lösung aus 29,5 g Natrium-n-propanolat (0,36 Mol) und 800 ml n-Propanol (10,6 Mol) wurden 50 g (0,31 Mol) 2-Naphthyl-mercaptan eingetragen. Das Gemisch erhitzte man in einem 1,2-l-Rührautoklaven mit einem Kohlenmonoxiddruck von 300 bar während 60 min von 25 auf 150°C und hielt das Gemisch dann während 10 h bei 150°C. Durch Destillation des Reaktoraustrags wurden 58,8 g (Ausbeute 95% der Theorie) 2-Naphthyl-n-propyl-thioether (Kp 115–120°C/0,6 mbar) erhalten.

Beispiele 3 bis 11

Die Herstellung der folgenden Thioether wurde analog Beispiel 1 durchgeführt, wobei aber statt

Mercaptan und Alkalialkoholat Alkalimercaptide verwendet wurden. Zusammensetzung des Ausgangsgemischs und die Ergebnisse sind in der Tabelle zusammengefasst.

Tabelle

| Beispiel | g Mercaptid II | Mercaptid $R^1$-S-M $R^1$ | M | g Alkohol III | Alkohol $R^2$-OH $R^2$ | Thioether $R^1$-S-$R^2$ Ausbeute in % der Theorie |
|---|---|---|---|---|---|---|
| 3 | 93 | 2-Naphthyl | K | 400 | Methyl | 98 |
| 4 | 29 | 2-Naphthyl | Na | 100 | Ethyl | 80 |
| 5 | 62 | 2-Naphthyl | K | 700 | Benzyl | 95 |
| 6 | 62 | 2-Naphthyl | K | 600 | n-Octyl | 91 |
| 7 | 36 | n-Dodecyl | K | 100 | Methyl | 81 |
| 8 | 70 | n-Dodecyl | Na | 650 | n-Propyl | 77 |
| 9 | 20 | 2-Naphthyl | Na | 120 | Isopropyl | 86 |
| 10 | 28 | 2-Naphthyl | Na | 120 | 2-Butyl | 85 |
| 11[a] | 30 | 2-Naphthyl | K | 140 | Cyclohexyl | 60 |

[a] Die Reaktionstemperatur betrug 180°C.

Beispiel 12

Eine Mischung aus 30 g Kalium-2-naphthyl-mercaptid und 120 g Methylformiat wurden bei Raumtemperatur in einen 0,3l-Rührautoklaven gefüllt und 15 h auf 150°C erhitzt. Dabei stellte sich ein Druck (Partialdruck von CO und Reaktionsgemisch) von 210 bar ein. Nach der Aufarbeitung analog Beispiel 1 erhielt man 23,5 g 2-Naphthylmethylthioether (89% der Theorie).

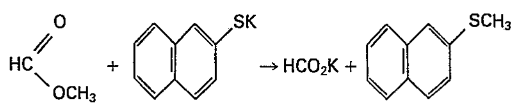

**Patentanspruch**

Verfahren zur Herstellung von Thioethern der Formel

$$R^1-S-R^2 \qquad I,$$

worin $R^1$ und $R^2$ gleich oder verschieden sein können und jeweils einen aliphatischen, cycloaliphatischen oder araliphatischen Rest bedeuten, $R^1$ auch einen aromatischen Rest bezeichnet, durch Umsetzung von Mercaptanen mit Sauerstoffverbindungen, dadurch gekennzeichnet, dass man Alkalimercaptide der Formel

$$R^1-S-M \qquad II,$$

worin $R^1$ die vorgenannte Bedeutung besitzt und M ein Alkaliatom bezeichnet,

a) mit Alkoholen der Formel

$$R^2-OH \qquad III,$$

worin $R^2$ die vorgenannte Bedeutung besitzt, und Kohlenmonoxid oder

b) mit Formiaten der Formel

$$H-\overset{\overset{O}{\|}}{C}-OR^2 \qquad IV,$$

worin $R^2$ die vorgenannte Bedeutung besitzt, umsetzt.

**Claim**

A process for the preparation of a thioether of the formula

$$R^1-S-R^2 \qquad I,$$

where $R^1$ and $R^2$ may be identical or different and are each an aliphatic, cycloaliphatic or araliphatic radical, and $R^1$ may furthermore be an aromatic radical, by reacting a mercaptan with an oxygen-containing compound, wherein an alkali metal mercaptide of the formula

$$R^1-S-M \qquad II,$$

where $R^1$ has the above meanings and M is an alkali metal atom, is reacted

a) with an alcohol of the formula

$$R^2-OH \qquad III,$$

where $R^2$ has the above meanings, and carbon monoxide, or

b) with a formate of the formula

$$H-\overset{\overset{O}{\|}}{C}-OR^2 \qquad IV,$$

where $R^2$ has the above meanings.

**Revendication**

Procédé de préparation de thio-éthers de la formule

$$R^1-S-R^2 \qquad I,$$

dans laquelle $R^1$ et $R^2$, qui peuvent être identiques ou différents, désignent chacun un groupe aliphatique, cycloaliphatique ou araliphatique, $R^1$ pouvant en outre être un groupe aromatique, par réaction de mercaptans avec des composés oxygénés, caractérisé en ce que l'on fait réagir des mercaptides de métaux alcalins de la formule

$$R^1-S-M \qquad II,$$

dans laquelle $R^1$ possède la signification définie et M désigne un atome de métal alcalin,

soit a) avec des alcools de la formule

$$R^2-OH \qquad III,$$

dans laquelle $R^2$ possède la signification définie, et de l'oxyde de carbone,

soit b) avec des formiates de la formule

$$H-\overset{\overset{\displaystyle O}{\|}}{C}-OR^2 \qquad IV,$$

dans laquelle $R^2$ possède la signification définie.